(19) Europäisches Patentamt
European Patent Office
Office européen des brevets

(11) Publication number: 0 124 287 B1

# (12) EUROPEAN PATENT SPECIFICATION

(45) Date of publication of patent specification: 03.03.93 (51) Int. Cl.⁵: C12Q 1/28

(21) Application number: 84302217.9

(22) Date of filing: 30.03.84

## (54) Method and test composition for determination of hydrogen peroxide.

(30) Priority: 31.03.83 JP 56652/83

(43) Date of publication of application:
07.11.84 Bulletin 84/45

(45) Publication of the grant of the patent:
03.03.93 Bulletin 93/09

(84) Designated Contracting States:
DE FR GB

(56) References cited:
EP-A- 0 038 205
EP-A- 0 045 220
US-A- 3 654 179

(73) Proprietor: Kyowa Medex Co. Ltd.
6-1 Ohtemachi Itchome Chiyoda-ku
Tokyo(JP)

(72) Inventor: Aoyama, Norihito
1188, Shimotogari Nagaizumi-cho
Sunto-gun Shizuoka-ken(JP)
Inventor: Miike, Akira
410-1 Nameri Nagaizumi-cho
Sunto-gun Shizuoka-ken(JP)
Inventor: Shimizu, Yoshiaki
127-5 Shimonagakubo Ikeda Nagaizumi-cho
Sunto-gun Shizuoka-ken(JP)
Inventor: Tatano, Toshio
2297-6 Ooka
Numazu-shi Shizuoka-ken(JP)

(74) Representative: Lambert, Hugh Richmond et al
D. YOUNG & CO. 10 Staple Inn
London, WC1V 7RD (GB)

EP 0 124 287 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method and test composition for the determination of hydrogen peroxide, and more particularly, to a method for the determination of hydrogen peroxide by reacting hydrogen peroxide with a novel chromogen as a hydrogen donor in the presence of peroxidase and determining the amount of pigment formed.

In the medical, diagnostic and biochemical arts, numerous substrates are determined by oxidizing the substrate with an oxidase and determining the amount of hydrogen peroxide produced. For example, cholesterol is determined by reaction with cholesterol oxidase and subsequent determination of the hydrogen peroxide produced, by reaction of the product hydrogen peroxide with a peroxidase in the presence of a chromogen which forms a pigment, and measuring the absorbancy of the reaction solution. In such processes, 4-aminoantipyrine (hereinafter referred to as "4AA") and phenol, 4AA and N,N-dimethylaniline, 4AA and N-ethyl-N($\beta$ -hydroxyethyl)-m-toluidine, 3-methylbenzothiazolin hydrazone and N,N-diethylaniline, 4AA and N-ethyl-N-(3-methylphenyl)-N'-acetylethylenediamine (hereinafter referred to as "EMAE") are commonly used as the chromogen or chromogenic mixture.

Also known for this purpose are the chromogens disclosed in EP-A-0 045 220, namely compounds of the formulae

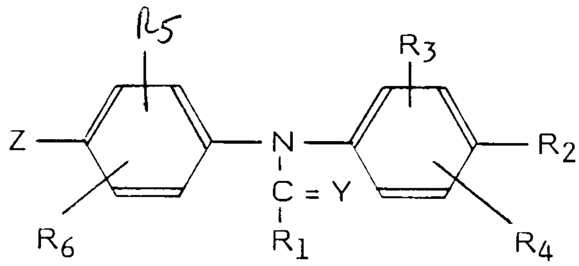

and

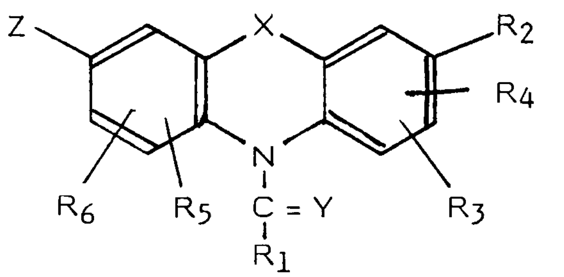

where Z represents hydroxyl, amino or substituted amino, Y represents an oxygen atom or sulfur atom, $R_1$ represents hydrogen, alkyl, alkenyl, aryl, amino or mono-substituted amino, $R_2$ represents hydrogen, hydroxyl, alkyl, alkenyl, aryl, amino, alkylamino, or alkoxy, $R_3$, $R_4$, $R_5$ and $R_6$ represent hydrogen, alkyl, alkenyl, acyl, aryl, halogen, sulfo, nitro, carboxyl, hydroxyl, hydroxyalkyl or alkoxy, $R_3$ and $R_4$ or $R_5$ and $R_6$ may form alkenylene, and X represents -S-, -O-,

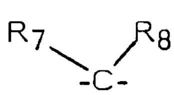

or

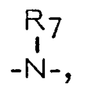

$R_7$ and $R_8$ represent hydrogen, alkyl, alkenyl or aryl.

In accordance with the present invention, similar chromogenic derivatives of diphenylamine, phenoxazin and phenothiazin have been found which are superior in sensitivity and which are not affected by components often found in vivo such as hemoglobin, bilirubin and uric acid.

These chromogens are compounds of the following formulae (I) and (II):

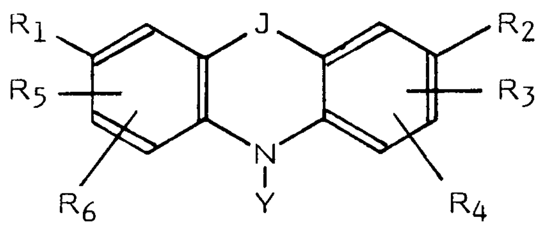

Formula I

and

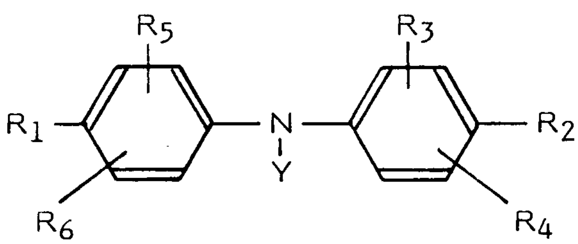

Formula II

where:

Y represents hydrogen or

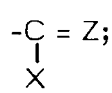

Z represents oxygen or sulfur;

X represents hydrogen, alkyl, alkenyl, aryl, aralkyl, amino or substituted amino;

$R_1$ represents hydroxyl, amino or substituted amino;

$R_2$ represents hydrogen, hydroxyl, alkyl, alkoxy, aryl, aralkyl, alkenyl, amino or substituted amino;

$R_3$ represents a group of formula (III), (IV), (V), (VI) or (VII) described hereinafter;

$R_4$, $R_5$ and $R_6$ are the same or different and represent hydrogen, alkyl, alkenyl, acyl, aryl, aralkyl, halogen atom, nitro, sulfo, carboxyl, hydroxyl, alkoxy or a group of formula (III), (IV), (V), (VI) or (VII) described hereinafter, with the proviso that $R_5$ and $R_6$ may also be joined together to form an alkenylene group;

J represents -S-, -O-, or a group of the formula

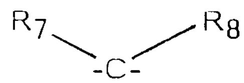

or

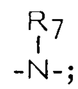

and

$R_7$ and $R_8$ are the same or different and represent hydrogen, alkyl or alkenyl.

Within the above definitions, the groups of formula III to VII are:

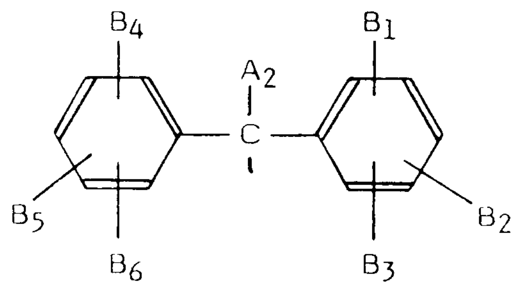

Formula III

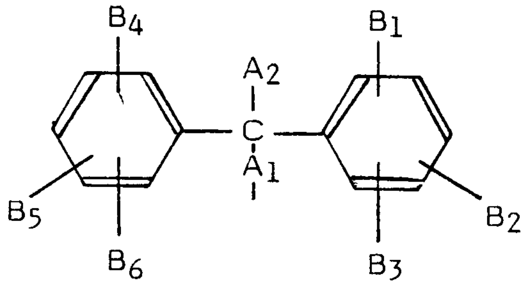

Formula IV

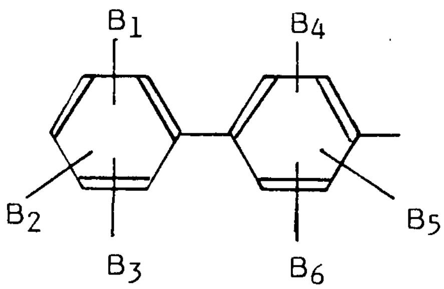

Formula V

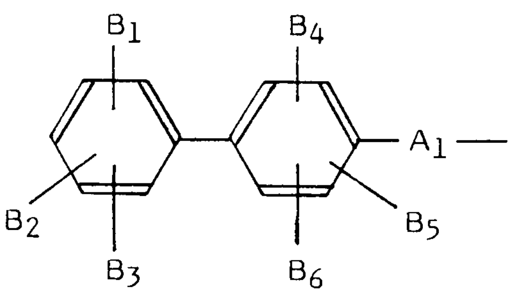

Formula VI

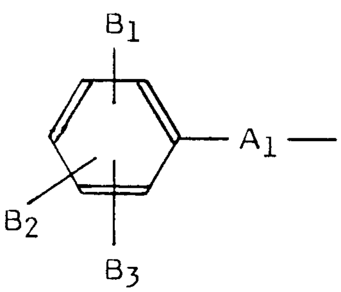

Formula VII

wherein:

$A_1$ represents alkylene;

$A_2$ has the same meaning as $R_2$;

$B_1$, $B_2$, $B_3$, $B_4$, $B_5$ and $B_6$ are the same or different and represent hydrogen, alkyl, alkenyl, acyl, aryl, aralkyl, halogen atom, nitro, sulfo, carboxyl, hydroxyl, alkoxy or hydroxyalkyl.

"Substituted amino" in the above definitions of $R_1$ and $R_2$, means amino substituted by alkyl, alkenyl, aryl, hydroxyalkyl, cycloalkyl, acyl, aralkyl, acylalkyl, carboxyl, alkoxy, sulfo or sulfoalkyl.

As used herein, alkyl includes alkyl having 1 to 5 carbon atoms, such as methyl, ethyl, propyl and butyl; alkenyl includes alkenyl having 2 to 5 carbon atoms such as vinyl, propylene and butylene; alkoxy includes alkoxy having 1 to 5 carbon atoms such as methoxy, ethoxy, propoxy and butoxy; aryl includes phenyl, naphthyl, and substituted phenyl; substituent of substituted phenyl includes alkyl having 1 to 4 carbon atoms, halogen such as chloro atom and bromo atom, amino, alkoxycarbonylamino, alkoxycarbonylaminoalkyl, alkoxy and acyl; substituted phenyl may have 1 to 5 substituents; aralkyl includes aralkyl having 7 to 10 carbon atoms such as benzyl; acyl includes acyl having 2 to 5 carbon atoms such as acetyl, propionyl and butyryl; halogen includes fluoro atom, chloro atom and bromo atom.

Alkenylene includes alkenylene having 3 to 4 carbon atoms such as -CH = CH-CH = CH-, -CH = CH-$CH_2$-, etc.

Also, where hereinafter reference is made to the numerical position of substituent groups on the aromatic ring structures, the following ring-numbering systems are used, viz:

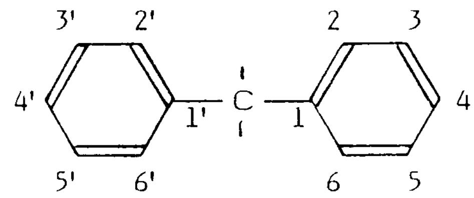

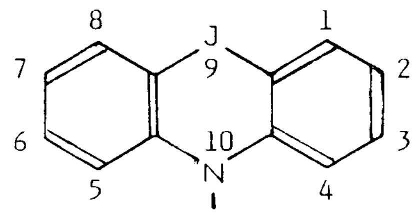

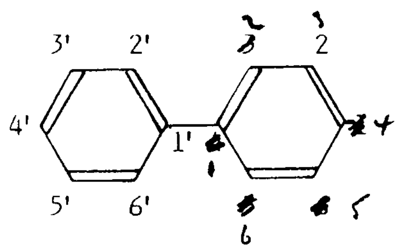

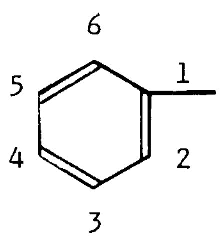

and

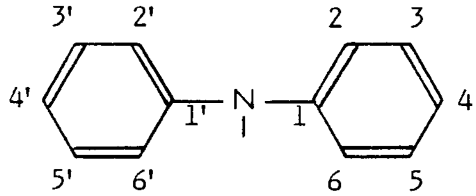

In utilising the chromogens of the present invention in the determination of hydrogen peroxide, the chromogen is added to a sample containing hydrogen peroxide or to the system where hydrogen peroxide is produced (hereinafter referred to as "$H_2O_2$-producing system") together with peroxidase. The absorbancy of the resultant coloured solution is measured at a wavelength in the visible or near infra-red ray region, e.g.

in the range 600 to 900 nm. The amount of hydrogen peroxide in the sample is then calculated by comparison with a standard curve.

The reaction is usually carried out at a temperature in the range 5 to 50 ° C, preferably 25 to 40 ° C, in a buffer solution having a pH of 2 to 10, and is usually completed within a few minutes.

The chromogen is preferably used in an amount of from 10 to 1000 mole equivalents relative to hydrogen peroxide. The peroxidase is used in a concentration of 0.1 to 1000 IU/ml.

As buffers, Good buffer, phosphate buffer, tris-HCl buffer, succinate buffer, citrate buffer, acetate buffer, etc. may be used, at concentrations of from 0.005 to 2 mol/l.

A small amount of phenol or EMAE can be added to the reaction system to activate the peroxidase and to promote pigment formation.

Examples of chromogens according to the present invention are shown in Tables 1 and 2, where the following codes apply:

P : Compound of formula I or II
M : $-CH_3$
E : $-C_2H_5$
D : $-CH_2CH_2CH_2SO_3H$
$\alpha_1$ : $-CH_2CH_2CH_3$
$\alpha_2$ : $>CHCH_2CH_2CH_3$

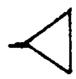 : 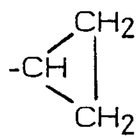

Ph : 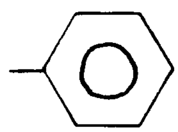

In each of compounds 1 to 34, $R_4$, $R_6$, $B_2$, $B_3$, $B_5$ and $B_6$ are all hydrogen. In compounds 1 to 27 and 31 to 34 (Table 1), Y is H. In compounds 28, 29 and 30 (Table 2), Y is

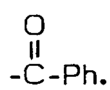

In compounds 18 and 19 (Table 1, compound formula I) J is -S-.

TABLE 1

| Compound No. | P | $R_1$ | $R_2$ | $R_3$ | $R_5$ | $A_1$ | $A_2$ | $B_1$ | $B_4$ |
|---|---|---|---|---|---|---|---|---|---|
| 1 | II | $NM_2$ | $NM_2$ | III | III | - | H | Cl | H |
| 2 | II | $NM_2$ | $NM_2$ | III | III | - | H | F | H |
| 3 | II | $NM_2$ | $NM_2$ | III | III | - | H | H | H |
| 4 | II | $NM_2$ | $NM_2$ | III | III | - | H | OM | OM |
| 5 | II | $NM_2$ | $NM_2$ | III | III | - | H | Cl | Cl |
| 6 | II | $NM_2$ | $NM_2$ | III | III | - | H | F | F |
| 7 | II | $NM_2$ | $NM_2$ | III | III | - | H | $NM_2$ | $NM_2$ |
| 8 | II | $NM_2$ | $NM_2$ | III | H | - | H | Cl | Cl |
| 9 | II | $NH_2$ | $NH_2$ | III | III | - | H | H | H |
| 10 | II | $NM_2$ | $NM_2$ | IV | H | $CH_2$ | H | Cl | Cl |
| 11 | II | $NM_2$ | $NM_2$ | IV | H | $CH_2$ | M | Cl | Cl |
| 12 | II | $NM_2$ | $NM_2$ | VI | VI | $CH_2$ | - | H | H |
| 13 | II | $NM_2$ | $NM_2$ | III | H | - | 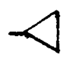 | H | H |
| 14 | II | $NM_2$ | $NM_2$ | III | H | - | $\alpha_1$ | H | H |

TABLE 1 (continued)

| Compound No. | P | $R_1$ | $R_2$ | $R_3$ | $R_5$ | $A_1$ | $A_2$ | $B_1$ | $B_4$ |
|---|---|---|---|---|---|---|---|---|---|
| 15 | II | $NM_2$ | $NM_2$ | VII | H | $\alpha_2$ | - | H | - |
| 16 | II | $NM_2$ | $NM_2$ | VII | VII | $CH_2$ | - | Cl | - |
| 17 | II | $NM_2$ | $NM_2$ | VII | VII | $CH_2$ | - | Br | - |
| 18 | I | $NM_2$ | $NM_2$ | III | H | - | H | Cl | Cl |
| 19 | I | $NM_2$ | $NM_2$ | III | $NO_2$ | - | H | Cl | Cl |
| 20 | II | $NH_2$ | OH | III | H | - | H | Cl | Cl |
| 21 | II | $NH_2$ | OM | III | H | - | H | Cl | Cl |
| 22 | II | $NH_2$ | OM | III | H | - | H | F | F |
| 23 | II | $NH_2$ | OH | III | H | - | H | F | F |
| 24 | II | $NH_2$ | $NH_2$ | III | III | - | H | Cl | Cl |
| 25 | II | $NH_2$ | $NH_2$ | III | III | - | H | F | F |
| 26 | II | $NH_2$ | $NH_2$ | III | III | - | H | H | Cl |
| 27 | II | $NH_2$ | $NH_2$ | III | III | - | H | H | F |
| 31 | II | $ND_2$ | $ND_2$ | III | III | - | H | H | H |
| 32 | II | $ND_2$ | $ND_2$ | III | III | - | H | Cl | Cl |
| 33 | II | NHD | NHD | III | III | - | H | H | H |
| 34 | II | NHD | NHD | III | III | - | H | Cl | Cl |

TABLE 2

| Compound No. | P | J | $R_1$ | $R_2$ | $R_3$ | $R_5$ | $A_2$ | $B_1$ | $B_4$ |
|---|---|---|---|---|---|---|---|---|---|
| 28 | II | - | $NM_2$ | $NM_2$ | III | III | H | Cl | Cl |
| 29 | I | S | $NM_2$ | $NM_2$ | III | H | H | Cl | Cl |
| 30 | I | O | $NE_2$ | $NE_2$ | III | H | H | Cl | Cl |

In the above Tables, in the compounds of Formula II substituent $B_1$ is at the 4'-position, except in compounds 16 and 17, where it is at the 4-position. Substituent $B_4$ is at the 4-position in all compounds.

The results of comparative tests between the compounds indicated in Tables 1 and 2 and previously known chromogens are given in Table 3. The test procedure was as follows:

A solution containing 0.1 mg/ml of the chromogen in Good buffer solution (pH 6.5) containing 10 U/ml peroxidase, 0.001 mg/ml phenol and 0.1 mg/ml Triton X-100, is prepared. 3 ml of the chromogen solution is then added to 20 $\mu$l of 10.33 mg/dl $H_2O_2$ solution and the mixture allowed to react.

The OD value of the reaction solution at $\lambda$max is then measured and expressed as a relative numerical value taking the OD obtained using 4AA-EMAE as the chromogen as 100.

The adverse effect of serum components such as bilirubin, cysteine and uric acid is measured by adding 20 $\mu$g/3 ml bilirubin, 20 $\mu$g/3 ml of crysteine or 10 $\mu$g/3 ml of uric acid to the sample. The symbol "±" means that the effect is 3 to 6%, "+" means that the effect is "6 to 20%, "++" means that the effect is more than 20%, and "-" means that the effect is less than 3%.

The degree of the colour in the reagent blank is given in comparison with that of the colour obtained by using Leuco Bindschedler's Green (LBG) as representative prior art chromogen. The following codes are used:

AA : very little colour;
A : some colour, but still low;
B : colouration equal to that of LBG.

Table 3

| Compound No. | λ max (nm) | S | F | | | RB |
|---|---|---|---|---|---|---|
| | | | B | CY | U | |
| 1 | 747 | 390 | ± | ± | ± | A |
| 2 | 740 | 395 | ± | ± | ± | A |
| 3 | 735 | 380 | - | - | - | AA |
| 4 | 700 | 150 | + | + | ± | AA |
| 5 | 755 | 400 | - | - | - | AA |
| 6 | 745 | 400 | - | - | - | AA |
| 7 | 520 | 360 | ± | ± | ± | AA |
| 8 | 750 | 350 | ± | ± | ± | A |
| 9 | 720 | 200 | ± | + | + | A |
| 10 | 728 | 400 | ± | + | + | A |
| 11 | 690 | 200 | ± | + | + | A |

Table 3 (Continued)

| Compound No. | λmax (nm) | S | F B | F CY | F U | RB |
|---|---|---|---|---|---|---|
| 12 | 733 | 410 | ± | + | ± | AA |
| 13 | 728 | 370 | ± | + | ± | A |
| 14 | 725 | 360 | ± | + | ± | A |
| 15 | 747 | 390 | ± | ± | ± | B |
| 16 | 737 | 380 | ± | + | ± | A |
| 17 | 735 | 370 | ± | ± | ± | A |
| 18 | 680 | 700 | ± | ± | ± | B |
| 19 | 670 | 400 | ± | ± | ± | B |
| 20 | 700 | 200 | ± | ± | ± | A |
| 21 | 690 | 160 | ± | ± | ± | A |
| 22 | 723 | 310 | ± | ± | ± | A |
| 23 | 715 | 150 | ± | ± | ± | A |
| 24 | 708 | 270 | - | - | - | AA |
| 25 | 700 | 270 | - | - | - | AA |
| 26 | 710 | 250 | - | ± | - | AA |
| 27 | 715 | 260 | - | ± | - | AA |
| 28 | 755 | 395 | ± | ± | ± | AA |
| 29 | 680 | 700 | ± | ± | ± | B |
| 30 | 685 | 690 | ± | ± | ± | B |
| 31 | 760 | 350 | - | - | - | AA |
| 32 | 770 | 350 | - | - | - | AA |
| 33 | 710 | 320 | - | - | - | A |
| 34 | 720 | 320 | - | - | - | A |
| $RE_1$ | 728 | 410 | + | + | + | B |
| $RE_1$ | 555 | 100 | ++ | ++ | ++ | A |

$RE_1$ : LBG
$RE_2$ : 4AA-EMAE
S : Sensitivity
F : Effect of components in serum
B : Bilirubin
CY : Cysteine
U : Uric acid
RB : Degree of colour development of reagent blank

The results show the better reactivity of the present chromogens as hydrogen donors and their relative insensitivity to contaminants such as bilirubin, cysteine and uric acid.

The chromogens of the present invention are obtained by the condensation reaction of Leuco bases, obtained by the reduction of diphenyl amines, thiazine or oxazine pigments, with a benzhydrol in the presence of sulfuric acid. The condensation reaction is carried out at 30 to 100 °C for 1 to 100 hours. The

reaction mixture is then added to an organic solvent such as n-octanol, capable of crystallizing out the desired chromogen. The crude crystals are then re-dissolved in an organic solvent, such as methanol, and the solution subjected to column chromatography through a silica gel column to recover the desired compound.

The preparation of the compounds listed in Tables 1 and 2 is illustrated below.

(1) Preparation of Compound Nos. 1 to 7, 12, 16 and 17

Bindschedler's Green Leuco base [4,4'-bis(dimethylamino)diphenylamine] (hereinafter referred to as "BGLB") was reacted with each of the undermentioned compounds in a molar ratio of 1:2 in the presence of an amount of 60% sulfuric acid equal to 5 times the amount of Leuco base. The reaction mixture was stirred at 80 ° C for 2 to 3 hours.

At the end of the reaction, n-octanol was added to crystallize the condensation product and the crude crystal product was dissolved in a small amount of methanol. The solution was then subjected to column chromatography in a silica gel column and eluted with a 2:1 mixture of chloroform and n-hexane. The fractions containing the purified product chromogen were collected and evaporated to dryness.

| Compound No. | Melting Point (° C) | Reactant |
|---|---|---|
| 1 | 102 - 105 | 4-chlorobenzhydrol |
| 2 | 98 - 101 | 4-fluorobenzhydrol |
| 3 | 120 - 123 | benzhydrol |
| 4 | 152 - 155 | 4,4'-dimethoxybenzhydrol |
| 5 | 184 - 187 | 4,4'-dichlorobenzhydrol |
| 6 | 111 - 114 | 4,4'-difluorobenzhydrol |
| 7 | 89 - 92 | 4,4'-dimethylaminobenzhydrol |
| 12 | 108 - 111 | 4-biphenylmethanol |
| 16 | 95 - 98 | p-chlorobenzylalcohol |
| 17 | 104 - 107 | p-bromobenzylalcohol |

(2) Preparation of Compound Nos. 8, 10, 11 and 13 to 15

The same procedure as described in (1) above was carried out except that the BGLB was reacted with the following compounds at a molar ratio of 10:1 at 50 ° C.

| Compound No. | Melting Point (° C) | Reactant |
|---|---|---|
| 8 | 78 - 81 | 4,4'-dichlorobenzhydrol |
| 10 | 131 - 133 | 2,2'-bis(p-chlorophenyl)-ethanol |
| 11 | 128 - 131 | 2,2'-bis(p-chlorophenyl)-propanol |
| 13 | 82 - 85 | cyclopropyl-benzhydrol |
| 14 | 84 - 87 | 1,1'-diphenylbutanol |
| 15 | 172 - 175 | 1-phenylbutanol |

(3) Preparation of Compound Nos. 9 and 24 to 27

Following the same general procedure as described in (1) above, 4,4'-diamino-diphenyl amine sulfate salt was reacted respectively with diphenylmethanol, 4,4'-dichlorobenzhydrol, 4,4'-difluorobenzhydrol, 4-chlorobenzhydrol and 4-fluorobenzhydrol. The resulting compounds 9 and 24 to 27 have melting points of 158 to 161 ° C, 173 to 175 ° C, 89 to 92 ° C, 155 to 158 ° C and 104 to 106 ° C respectively.

(4) Preparation of Compound Nos. 18 and 19

Following the same general procedure as described in (1) above, reduction reaction products of Methylene Blue (C.I. 52015) and Methylene Green (C.I. 52020) respectively were reacted with 4,4'-dichlorobenzhydrol at a molar ratio of 10:1 and at 50°C. The resulting compounds have melting points of 179 to 180°C and 148 to 151°C respectively.

(5) Preparation of Compound No. 29

Following the same general procedure as described in (1) above, benzoyl Leuco Methylene Blue was reacted with 4,4'-dichlorobenzhydrol in a molar ratio of 10:1 at 50°C to obtain compound No. 29 (m.p. 212 to 215°C).

(6) Preparation of Compound No. 28

Compound No. 5, prepared as above, was reacted with phenyl isocyanate in the presence of dimethylformamide (DMF) at 50°C for 3 days to obtain compound No. 28 (m.p. 196 to 200°C).

(7) Preparation of Compound No. 30

Basic Blue (C.I. 51004) was reduced using sodium borohydride and the product reacted with 4,4,-dichlorobenzhydrol in a molar ratio of 10:1 in 60% sulfuric acid at 50°C. The condensation product was purified by the same purification procedures as described in (1) above, and the purified product reacted with an equal molar amount of isocyanic acid in the presence of DMF at 50°C for 3 days. The purification procedures as described in (1) above were repeated to obtain compound No. 30 (m.p. 118 to 120°C).

(8) Preparation of Compound Nos. 20 and 23

Following the same general procedure as described in (1) above, p-(p-aminoanilino)-phenol was reacted respectively with 4,4'-dichlorobenzhydrol and 4,4'-difluorobenzhydrol at a molar ratio of 10:1 in 60% sulfuric acid at 50°C. The product compound Nos. 20 and 23 have m.p. 89 to 91°C and 121 to 123°C respectively.

(9) Preparation of Compound Nos. 21 and 22

Following the same general procedure as described in (1) above, Variamine Blue hydrochloride was reacted respectively with 4,4'-dichlorobenzhydrol and 4,4'-difluorobenzhydrol at a molar ratio of 10:1 in 60% sulfuric acid at 50°C. The product compound Nos. 21 and 22 respectively have m.p. 163 to 165°C and 158 to 160°C.

(10) Preparation of Compound Nos. 31 to 34

4,4'-diamino-diphenylamine sulfate and propane sultone in a molar ratio of 1:20 were added to chloroform and the mixture stirred at 50°C for 5 to 7 days. Distilled water was then added to the reaction mixture in equal amount. After vigorous stirring, the water layer was separated and evaporated to dryness. The dried product was then reacted with benzhydrol and 4,4'-dichlorobenzhydrol, respectively, in a weight ratio of 2:1 in 60% sulfuric acid. The reaction mixture was stirred at 80°C for 2 to 3 hours.

The same purification procedures as described in (1) above were followed except that different polarity solvents were used following initiation of the elution with chloroform. Compound Nos. 33 and 34 are obtained using solvents of low polarity. Compounds Nos. 31 and 32 are subsequently eluted using solvents of higher polarity.

m.p., Compound No. 31 : 93 to 95°C
m.p., Compound No. 32 : 78 to 82°C
m.p., Compound No. 33 : 62 to 65°C
m.p., Compound No. 34 : 45 to 48°C

The chromogens of the present invention are useful in the determination of substrates or enzymatic activity in the $H_2O_2$-producing system. In such systems both the $H_2O_2$-producing reaction and the determination of the product $H_2O_2$ can proceed at the same time, thus providing a simple and convenient system for the determination of enzyme activity or substrate concentration. Typical examples of such enzyme/substrate systems are the following combinations: uric acid-uricase, cholesterol-cholesterol oxidase, cholesterol ester-cholesterol esterase and cholesterol oxidase, xanthin, hypoxanthin or guanine-xanthin oxidase, phospholipase D-lecithin-choline oxidase, choline-choline oxidase, pyruvic acid-pyruvate oxidase-phosphoric acid, triglyceride-lipoprotein lipase-ATP-glycerin kinase-glycerin-3-phosphate oxidase, fatty acid-coenzyme A-acyl Co A synthetase-acyl Co A oxidase, triglyceride-lipase-glycerol oxidase, glucose-glucose oxidase and galactose-galactose oxidase.

The substrates of these enzymatic reactions are contained in serum, urine, etc., and the determination of the substrates is useful for diagnostic purposes.

Examples of the enzymatic reactions involved are schematically shown as follows.

1. Uric acid

$$\text{Uric acid} + O_2 + 2H_2O \xrightarrow{\text{Uricase}} \text{Allantoin} + CO_2 + H_2O_2$$

2. Total cholesterol

$$\text{Cholesterol ester} + H_2O \xrightarrow[]{\text{Cholesterol esterase}} \text{Free form of cholesterol} + \text{Fatty acid}$$

$$\text{Free form of cholesterol} + O_2 \xrightarrow{\text{Cholesterol oxidase}} \text{Cholesterone} + H_2O_2$$

3. Triglyceride

$$\text{Triglyceride} + 3H_2O \xrightarrow{\text{Lipoprotein lipase}} \text{Glycerol} + \text{Fatty acid}$$

$$\text{Glycerol} + O_2 \xrightarrow{\text{Glycerol oxidase}} \text{Glyceraldehyde} + H_2O_2$$

4. Free form of fatty acid

$$\text{Free form of fatty acid} + \text{ATP} + \text{CoA} \xrightarrow{\text{Acyl-CoA synthetase}} \text{Acyl-CoA} + \text{AMP} + \text{PPi}$$

$$\text{Acyl-CoA} + O_2 \xrightarrow{\text{Acyl-CoA oxidase}} \text{Trans-2,3-dehydroacyl-CoA} + H_2O_2$$

5. Sialic acid

$$\text{Sialic acid (bound type)} \xrightarrow{\text{Neuraminidase}} \text{N-acetyl neuraminic acid (NANA)}$$

$$\text{NANA} \xrightarrow{\text{NANA-aldolase}} \text{N-acetylmannosamine} + \text{Pyruvic acid}$$

$$\text{Pyruvic acid} + O_2 + \text{Phosphoric acid} \xrightarrow{\text{Pyruvate oxidase, thiamine pyrophosphoric acid, Mg}} H_2O_2 + \text{Acetylphosphoric acid} + CO_2$$

6. Pyruvic acid

$$\text{Pyruvic acid} + O_2 + \text{Phosphoric acid} \xrightarrow[]{\text{Pyruvate oxidase, thiamine pyrophosphoric acid, Mg}} H_2O_2 + \text{Acetylphosphoric acid} + CO_2$$

7. Glucose

$$\text{Glucose} + O_2 \xrightarrow{\text{Pyranose oxidase}} \text{D-gluconolactone} + H_2O_2$$

8. Inorganic phosphorus

$$HPO_4^{--} + \text{Inosine} \xrightarrow{\text{Purine nucleotide phosphorylase}} \text{Hypoxanthine} + \text{ribose-1-phosphoric acid}$$

$$\text{Hypoxanthine} + 2H_2O + O_2 \xrightarrow{\text{Xanthine oxidase}} \text{Uric acid} + 2H_2O_2$$

9. Phospholipid

$$\text{Lecithin} \xrightarrow{\text{Phospholipase D}} \text{Choline} + \text{Phosphatidyl acid}$$

$$\text{Choline} \xrightarrow{\text{Choline oxidase}} 2H_2O_2 + \text{Betaine}$$

10. Monoamine oxidase

$$\text{Monoamine} + O_2 + H_2O \xrightarrow{\text{Monoamine oxidase}} \text{Acrolein} + NH_3 + H_2O_2$$

11. Choline esterase

$$\text{o-Toluoylcholine} + H_2O \xrightarrow{\text{Choline esterase}} \text{o-Toluoyl acid} + \text{Choline}$$

$$\text{Choline} + 2O_2 + H_2O \xrightarrow{\text{Choline oxidase}} \text{Betaine} + 2H_2O_2$$

The hydrogen peroxide-producing reaction and pigment-producing reaction may be conducted stepwise or more preferably, simultaneously by adding to the sample all the components necessary for the determination of hydrogen peroxide and conducting all the reactions in one step, and measuring the absorbancy of the reaction solution. For this purpose, the various components include the oxidase for the substrate to be determined, a peroxidase, and the chromogen (I) or (II). A buffer solution and surfactant, etc., may be added, if necessary. Of course, if other components are necessary in addition to the oxidase for oxidising the substrate, then these must he added to the $H_2O_2$-producing system.

The present invention also provides a test composition for the determination of hydrogen peroxide comprising a peroxidase and a chromogen represented by the formula (I) or (II). The composition may also contain a suitable buffer as well as surfactants such as polyoxyethylenealkylether, antiseptics such as sodium azide, and ascorbate oxidase for decomposing ascorbic acid. Also included are such test compositions which additionally contain a second enzyme, e.g. an oxidase which is capable of producing hydrogen peroxide in situ in the sample from, and proportional to, the concentration of a substrate for said second

enzyme in the said sample, thus enabling the quantitative determination of said substrate in said sample via the determination of the $H_2O_2$ produced in the sample by the second enzyme.

Example 1

In this Example 18 U of uricase, 100 U of peroxidase, 100 mg of Triton X-100, 0.1 mg of phenol and (A) 100 mg of 4AA and 100 mg of EMAE, (B) 50 mg of LBG, (C) 22.5 mg of Compound No.3, (D) 22.5 mg of Compound No. 5, (E) 22.5 mg of Compound No. 6, (F) 22.5 mg of Compound No. 32 or (G) 22.5 mg of Compound No. 33, are dissolved in 100 ml of 50 mM Good buffer solution (pH 6.5) to prepare a reagent solution. 20 µl of serum and 3 ml of the reagent solution are mixed in a test tube and the mixture incubated at 37° C for 10 minutes.

The absorbancy of each reaction solution at λmax is measured using blank reagent as a control. The concentration of uric acid in the serum is calculated by using calibration curve prepared in advance.

For comparison, the same test sample is analysed by ultra violet spectrophotometry. The results are shown in Table 4.

TABLE 4

| Serum No. | Control | Uric acid content (mg/dl) | | | | | | |
|---|---|---|---|---|---|---|---|---|
| | | A | B | C | D | E | F | G |
| 1 | 5.7 | 5.9 | 5.0 | 5.7 | 5.6 | 5.7 | 5.7 | 5.6 |
| 2 | 2.8 | 3.8 | 4.0 | 2.9 | 2.7 | 2.7 | 2.7 | 2.8 |
| 3 | 11.0 | 9.2 | 12.8 | 11.0 | 11.0 | 11.0 | 11.1 | 11.1 |
| 4 | 4.0 | 5.8 | 4.9 | 4.1 | 4.1 | 3.9 | 4.0 | 3.9 |
| 5 | 6.2 | 6.5 | 6.5 | 6.1 | 6.1 | 6.3 | 6.1 | 6.0 |

Example 2

In this Example, 15 U of cholesterol oxidase, 20 U of cholesterol esterase, 150 U of peroxidase, 100 mg of Triton X-100, 0.1 mg of phenol and the chromogens (A to G) in Example 1, are dissolved in 100 ml of 50 mM Good buffer solution (pH 5.8) to prepare a reagent solution. 10 µl of serum and 3 ml of the reagent solution are poured into a test tube and the mixture incubated at 37° C for 10 minutes. The absorbancy of the reaction solution at λmax is measured using blank reagent as a control. The concentration of cholesterol in serum is calculated by using a calibration curve prepared in advance.

For comparison, the same sample is analysed by gas chromatography (GC). The results are shown in Table 5.

TABLE 5

| Serum No. | GC | Cholesterol content (mg/dl) | | | | | | |
|---|---|---|---|---|---|---|---|---|
| | | A | B | C | D | E | F | G |
| 1 | 92 | 82 | 102 | 90 | 91 | 89 | 89 | 93 |
| 2 | 243 | 248 | 280 | 240 | 242 | 242 | 241 | 242 |
| 3 | 100 | 90 | 115 | 99 | 100 | 98 | 100 | 100 |
| 4 | 178 | 169 | 193 | 178 | 176 | 179 | 177 | 178 |
| 5 | 112 | 120 | 140 | 111 | 111 | 112 | 111 | 112 |

Example 3

In this Example 9 U of acyl CoA synthetase, 57 U of acyl-CoA oxidase, 833 U of peroxidase, 200 mg of ATP, 27 mg of CoA, 70 mg of $MgCl_2 \cdot 6H_2O$, 100 mg of Triton X-100, 0.1 mg of phenol and the chromogens (A to G) used in Example 1, are dissolved in 100 ml of 0.1 M Good buffer solution (pH 6.75) to prepare a

reagent solution. To a 20 µl serum sample is added 3 ml of the reagent solution and the mixture heated to 37° C for 10 minutes. The absorbancy of the reaction solution at λmax is measured using blank reagent as a control and the concentration of free fatty acid in serum is calculated from a calibration curve. For comparison, the same samples are analysed by gas chromatography (GC). The results are shown in Table 6.

TABLE 6

| Serum No. | GC | Free fatty acid content (mg/dl) | | | | | | |
|---|---|---|---|---|---|---|---|---|
| | | A | B | C | D | E | F | G |
| 1 | 80 | 53 | 102 | 82 | 80 | 79 | 79 | 81 |
| 2 | 115 | 180 | 160 | 115 | 118 | 116 | 114 | 115 |
| 3 | 102 | 42 | 280 | 101 | 100 | 101 | 102 | 101 |
| 4 | 258 | 290 | 295 | 260 | 261 | 259 | 259 | 257 |
| 5 | 180 | 300 | 220 | 180 | 179 | 179 | 181 | 179 |

Example 4

In this Example, 100 ml of Good buffer solution (pH 6.0) containing 140 mg of potassium dihydrogen phosphate, 23 mg of thiamine pyrophosphoric acid, 27 mg of $MgCl \cdot 6H_2O$, 300 U of pyruvate oxidase, 100 U of peroxidase, 10 mg of Triton X-100, 0.1 mg of phenol and (A) 100 mg of 4AA and 100 mg of EMAE, (B) 50 mg of LBG, (C) 22.5 mg of Compound No. 5, (D) 22.5 mg of Compound No. 24, (E) 22.5 mg of Compound No. 25, (F) 22.5 mg of Compound No. 26 or (G) 22.5 mg of Compound No. 27, is prepared as a reagent solution. To a 50 µl serum sample is added 3 ml of the reagent solution and the mixture reacted at 37° C for 10 minutes. The absorbancy of the reaction solution at λmax is measured using blank reagent as a control.

The concentration of pyruvate in the sample is calculated from a calibration curve and the results are shown in Table 7. For comparison, the same sample is analysed by a UV method using lactate dehydrogenase.

TABLE 7

| Serum No. | UV | Pyruvic acid content (mg/dl) | | | | | | |
|---|---|---|---|---|---|---|---|---|
| | | A | B | C | D | E | F | G |
| 1 | 0.38 | 0.53 | 0.69 | 0.38 | 0.38 | 0.36 | 0.37 | 0.38 |
| 2 | 0.91 | 1.81 | 0.76 | 0.93 | 0.90 | 0.91 | 0.91 | 0.89 |
| 3 | 0.55 | 0.22 | 1.10 | 0.57 | 0.53 | 0.57 | 0.55 | 0.53 |
| 4 | 0.72 | 1.01 | 1.31 | 0.70 | 0.71 | 0.70 | 0.74 | 0.70 |
| 5 | 0.50 | 0.63 | 0.33 | 0.49 | 0.49 | 0.50 | 0.48 | 0.52 |

## Claims

**1.** A method for the determination of hydrogen peroxide in a sample which comprises reacting the hydrogen peroxide with a peroxidase in the presence of a chromogen and measuring the absorbancy of the reaction solution in the visible or near infra-red region of the spectrum, characterised in that the chromogen is a compound of the formula

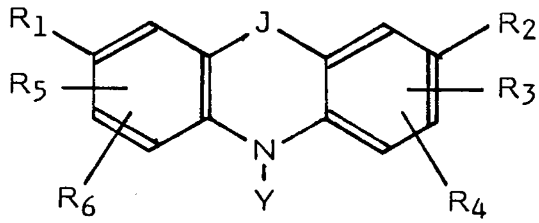

or

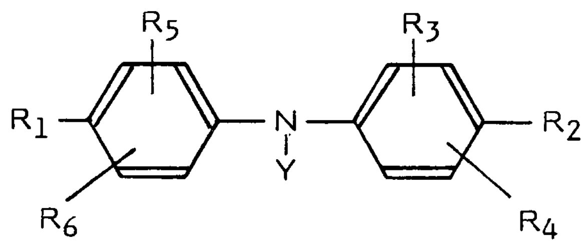

where:

Y represents hydrogen or

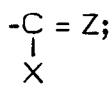

Z represents oxygen or sulfur;

X represents hydrogen, alkyl, alkenyl, aryl, aralkyl, amino or substituted amino;

$R_1$ represents hydroxyl, amino or substituted amino;

$R_2$ represents hydrogen, hydroxyl, alkyl, alkoxy, aryl, aralkyl, alkenyl, amino or substituted amino;

$R_3$ represents a group of formula (III), (IV), (V), (VI) or (VII) described hereinafter;

$R_4$, $R_5$ and $R_6$ are the same or different and represent hydrogen, alkyl, alkenyl, acyl, aryl, aralkyl, halogen atom, nitro, sulfo, carboxyl, hydroxyl, alkoxy or a group of formula (III), (IV), (V), (VI) or (VII) described hereinafter, with the proviso that $R_5$ and $R_6$ may also be joined together to form an alkenylene group;

J represents -S-, -O-, or a group of the formula

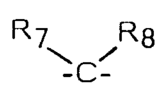

or

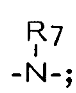

and

$R_7$ and $R_8$ are the same or different and represent hydrogen, alkyl or alkenyl;

the groups of formula III to VII being:

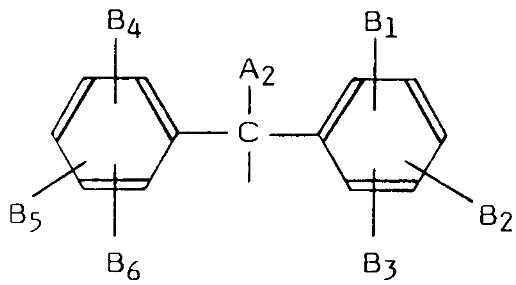

Formula III

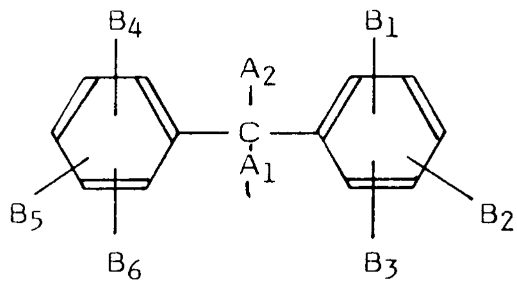

Formula IV

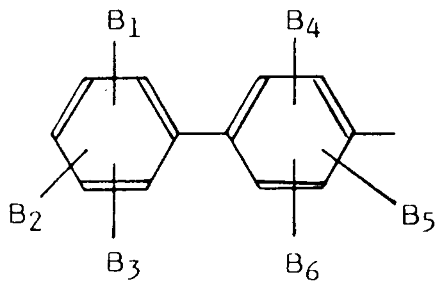

Formula V

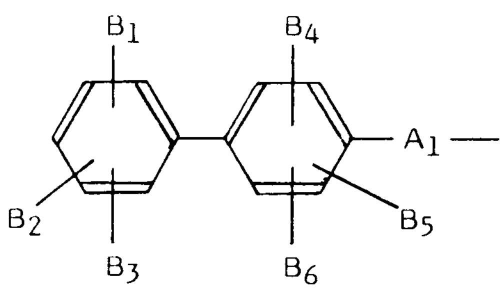

Formula VI

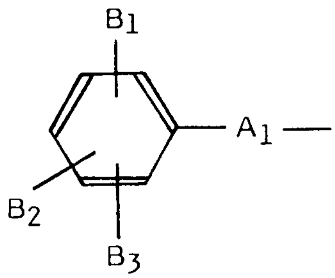

Formula VII

wherein:

$A_1$ represents alkylene;

$A_2$ has the same meaning as $R_2$;

$B_1$, $B_2$, $B_3$, $B_4$, $B_5$ and $B_6$ are the same or different and represent hydrogen, alkyl, alkenyl, acyl, aryl, aralkyl, halogen atom, nitro, sulfo, carboxyl, hydroxyl, alkoxy or hydroxyalkyl.

**2.** A method according to claim 1, wherein the hydrogen peroxide is produced in said sample as the result of an enzymatic reaction.

**3.** A method according to claim 2, wherein the hydrogen peroxide is produced in said sample as the result of an enzymatic reaction involving the reaction of an oxidase.

**4.** A method according to claim 3, wherein said oxidase is uricase, cholesterol oxidase, xanthine oxidase, choline oxidase, pyruvate oxidase, glycerin-3-phosphate oxidase, acyl CoA oxidase, glycerol oxidase, glucose oxidase or galactose oxidase.

**5.** A method according to claim 2, 3 or 4, wherein the hydrogen peroxide-producing enzymatic reaction and the reaction of hydrogen peroxide with said chromogen and the peroxidase are conducted simultaneously.

**6.** A method according to any one of claims 1 to 5, wherein the reaction(s) is or are carried out in a buffer solution.

**7.** A test composition for the determination of hydrogen peroxide, comprising a chromogen of formula I or II as defined in claim 1, and a peroxidase.

**8.** A test composition according to claim 7, which additionally contains a buffer.

**9.** A test composition according to claim 7 or 8, additionally containing a surfactant, antiseptic or ascorbate oxidase.

**10.** A test composition according to claim 7, 8 or 9, additionally containing a second enzyme capable of producing hydrogen peroxide in situ in the sample from, and proportional to the concentration of a substrate for said second enzyme in the said sample.

**11.** A test composition according to claim 10, wherein said second enzyme is an oxidase.

**12.** A test composition according to claim 11, wherein said oxidase is an oxidase as listed in claim 4.

**Patentansprüche**

**1.** Verfahren zur Bestimmung von Wasserstoffperoxid in einer Probe, das das Umsetzen des Wasserstoffperoxids mit einer Peroxidase in Gegenwart eines Chromogens und die Messung der Absorption der Reaktionslösung im sichtbaren oder nahen infraroten Spektralbereich umfaßt, dadurch gekennzeichnet, daß das Chromogen eine Verbindung der Formel

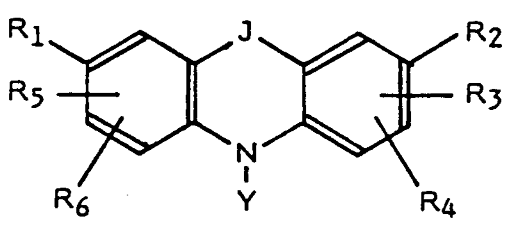

oder

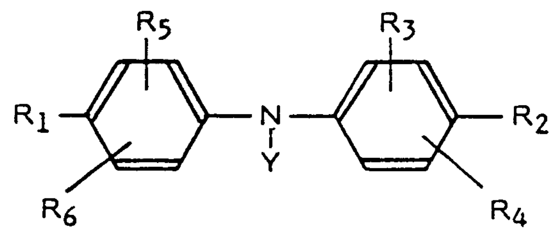

ist, wobei
Y ein Wasserstoffatom oder der

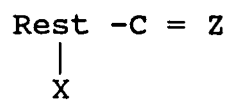

ist;
Z ein Sauerstoff- oder Schwefelatom ist;
X ein Wasserstoffatom, ein Alkyl-, Alkenyl-, Aryl-, Aralkyl-, Amino- oder substituierter Aminorest ist;
$R_1$ ein Hydroxyl-, Amino- oder substituierter Aminorest ist;
$R_2$ ein Wasserstoffatom, ein Hydroxyl-, ein Alkyl-, Alkoxy-, Aryl-, Aralkyl-, Alkenyl-, Amino-, oder substituierter Aminorest ist;
$R_3$ eine Gruppe der Formel (III), (IV), (V), (VI) oder (VII) die nachstehend beschrieben sind, ist;
$R_4$, $R_5$ und $R_6$ gleich oder verschieden sind und ein Wasserstoffatom, einen Alkyl-, Alkenyl-, Acyl-, Aryl-, Aralkylrest, ein Halogenatom, eine Nitro-, Sulfo-, Carboxyl-, Hydroxylgruppe, einen Alkoxyrest oder eine Gruppe der Formel (III), (IV), (V), (VI) oder (VII), die nachstehend beschrieben sind, darstellen, mit der Maßgabe, daß $R_5$ und $R_6$ auch miteinander zu einem Alkenylenrest verbunden sein können;
J ein Schwefel- oder Sauerstoffatom oder eine Gruppe der Formel

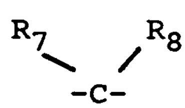

oder

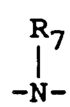

ist und
$R_7$ und $R_8$ gleich oder verschieden sind und ein Wasserstoffatom, einen Alkyl- oder Alkenylrest darstellten;
die Gruppen der Formel III bis VII

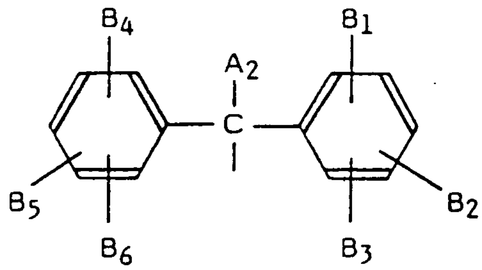

Formel III

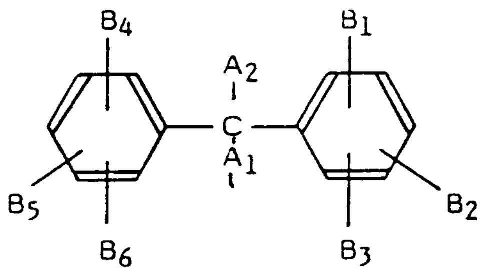

Formel IV

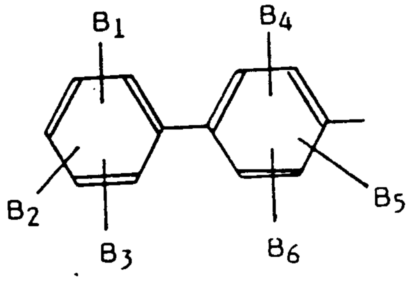

Formel V

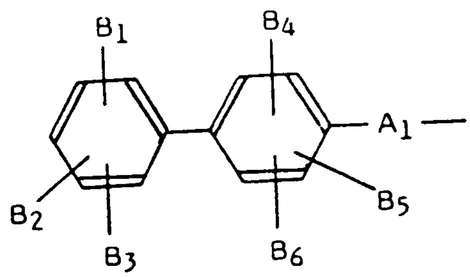

Formel VI

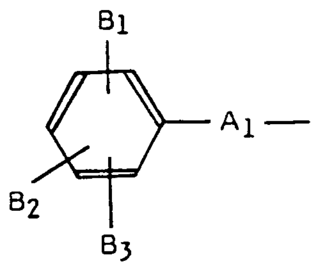

Formel VII

sind, wobei
$A_1$ ein Alkylenrest ist;
$A_2$ die gleiche Bedeutung wie $R_2$ hat;
$B_1$, $B_2$, $B_3$, $B_4$, $B_5$ und $B_6$ gleich oder verschieden sind und ein Wasserstoffatom, einen Alkyl-, Alkenyl-, Acyl-, Aryl-, Aralkylrest, ein Halogenatom, Nitro-, Sulfo-, Carboxyl-, Hydroxylgruppen, Alkoxy- oder Hydroryalkylreste sind.

**2.** Verfahren nach Anspruch 1, wobei das Wasserstoffperoxid in der Probe als Ergebnis einer enzymatischen Reaktion hergestellt wird.

**3.** Verfahren nach Anspruch 2, wobei das Wasserstoffperoxid in der Probe als Ergebnis einer enzymatischen Reaktion unter Beteiligung der Reaktion einer Oxidase hergestellt wird.

**4.** Verfahren nach Anspruch 3, wobei die Oxidase Uricase, Cholesterin-Oxidase, Xanthin-Oxidase, Cholin-Oxidase, Pyruvat-Oxidase, Glycerin-3-phosphat-Oxidase, Acyl-CoA-Oxidase, Glycerin-Oxidase, Glucose-Oxidase oder Galactose-Oxidase ist.

**5.** Verfahren nach Anspruch 2, 3 oder 4, wobei die Wasserstoffperoxid erzeugende enzymatische Reaktion und die Reaktion des Wasserstoffperoxids mit dem Chromogen und der Peroxidase gleichzeitig durchgeführt werden.

6. Verfahren nach einem der Ansprüche 1 bis 5, wobei die Reaktion(en) in einer Pufferlösung ausgeführt wird (werden).

7. Prüfzusammensetzung zur Bestimmung von Wasserstoffperoxid, umfassend ein Chromogen der Formel I oder II nach Anspruch 1 und eine Peroxidase.

8. Prüfzusammensetzung nach Anspruch 7, die zusätzlich einen Puffer enthält.

9. Prüfzusammensetzung nach Anspruch 7 oder 8, die zusätzlich ein Tensid, ein Antiseptikum oder Ascorbat-oxidase enthält.

10. Prüfzusammensetzung nach Anspruch 7, 8 oder 9, die zusätzlich ein zweites Enzym enthält, das dazu fähig ist, in der Probe in situ Wasserstoffperoxid aus einem Substrat für dieses zweite Enzym und proportional zur Konzentration dieses Substrats herzustellen.

11. Prüfzusammensetzung nach Anspruch 10, wobei das zweite Enzym eine Oxidase ist.

12. Prüfzusammensetzung nach Anspruch 11, wobei die Oxidase eine der in Anspruch 4 aufgeführten Oxidasen ist.

## Revendications

1. Procédé de dosage du peroxyde d'hydrogène dans un échantillon, qui comporte la réaction du peroxyde d'hydrogène avec une peroxydase en présence d'un chromogène, et la mesure de l'absorbance de la solution réactionnelle dans le domaine visible ou proche infrarouge du spectre, caractérisé en ce que le chromogène est un composé de formule :

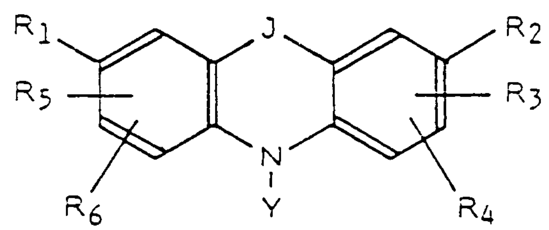

ou

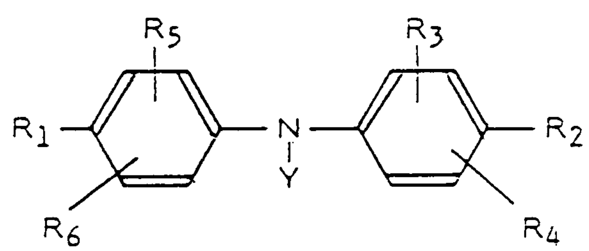

dans lesquelles :
Y représente un atome d'hydrogène ou un groupe -C(=Z)X, où Z représente un atome d'oxygène ou de soufre et X représente un atome d'hydrogène ou un groupe alkyle, alcényle, aryle, aralkyle, amino ou amino substitué ;
$R_1$ représente un groupe hydroxyle, amino ou amino substitué ;
$R_2$ représente un atome d'hydrogène ou un groupe hydroxyle, alkyle, alcoxy, aryle, aralkyle, alcényle, amino ou amino substitué ;
$R_3$ représente un groupe de formule (III), (IV), (V), (VI) ou (VII), formules qui sont décrites plus loin ;
$R_4$, $R_5$ et $R_6$ sont identiques ou différents et représentent des atomes d'hydrogène ou d'halogène ou

des groupes alkyle, alcényle, acyle, aryle, aralkyle, nitro, sulfo, carboxyle, hydroxyle ou alcoxy, ou encore des groupes de formule (III), (IV), (V), (VI) ou (VII), formules qui seront décrites plus loin, $R_5$ et $R_6$ pouvant également être réunis l'un à l'autre pour former un groupe alcénylène ;
J représente -S-, -O- ou un groupe de formule $-CR_7R_8-$ ou $-NR_7-$, $R_7$ et $R_8$ étant identiques ou différents et représentant des atomes d'hydrogène ou des groupes alkyle ou alcényle ;
les groupes de formules (III) à (VII) étant les suivants :

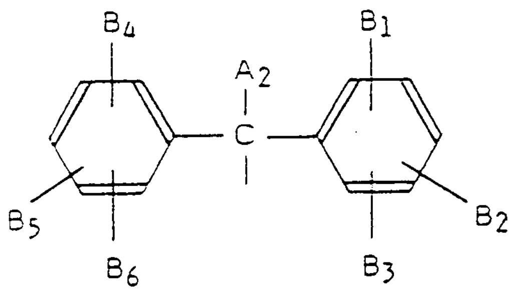

Formule III

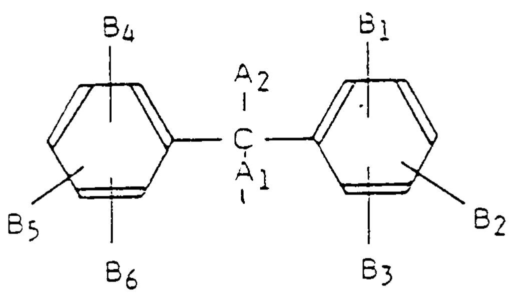

Formule IV

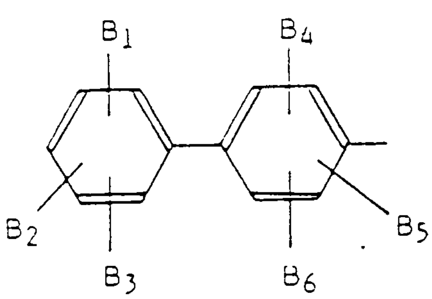

Formule V

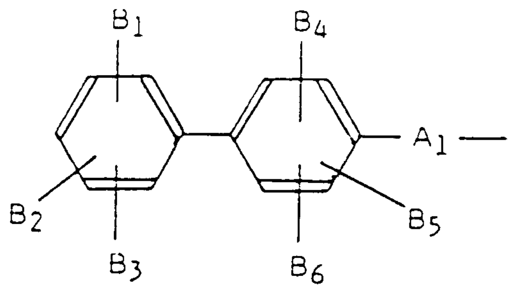

Formule VI

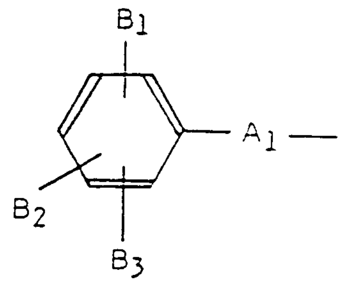

Formule VII

dans lesquelles formules :
$A_1$ représente un groupe alkylène ;
$A_2$ a la même signification que $R_2$ ;
$B_1$, $B_2$, $B_3$, $B_4$, $B_5$ et $B_6$ sont identiques ou différents et représentent des atomes d'hydrogène ou d'halogène ou des groupes alkyle, alcényle, acyle, aryle, aralkyle, nitro, sulfo, carboxyle, hydroxyle, alcoxy ou hydroxyalkyle.

**2.** Procédé conforme à la revendication 1, dans lequel le peroxyde d'hydrogène est produit dans ledit échantillon en résultat d'une réaction enzymatique.

**3.** Procédé conforme à la revendication 2, dans lequel le peroxyde d'hydrogène est produit dans ledit échantillon en résultat d'une réaction enzymatique impliquant la réaction d'une oxydase.

**4.** Procédé conforme à la revendication 3, dans lequel ladite oxydase est une uricase, une cholestérol oxydase, une xanthine oxydase, une choline oxydase, une pyruvate oxydase, une glycérine-3-phosphate oxydase, une acyl-CoA oxydase, une glycérol oxydase, une glucose oxydase ou une galactose oxydase.

**5.** Procédé conforme à la revendication 2, 3 ou 4, dans lequel la réaction enzymatique produisant le peroxyde d'hydrogène et la réaction du peroxyde d'hydrogène avec ledit chromogène et la peroxydase sont effectuées simultanément.

**6.** Procédé conforme à l'une quelconque des revendications 1 à 5, dans lequel la ou les réaction(s) est ou sont effectuée(s) dans une solution tampon.

**7.** Composition de test pour le dosage du peroxyde d'hydrogène, comprenant un chromogène de formule I ou II définie dans la revendication 1, et une peroxydase.

**8.** Composition de test conforme à la revendication 7, qui contient en outre un tampon.

**9.** Composition de test conforme à la revendication 7 ou 8, qui contient en outre en tensioactif, un antiseptique ou une ascorbate oxydase.

**10.** Composition de test conforme à la revendication 7, 8 ou 9, qui contient en outre une seconde enzyme capable de produire du peroxyde d'hydrogène in situ dans l'échantillon, à partir d'un substrat pour

cette seconde enzyme présent dans l'échantillon et en proportion de la concentration de ce substrat.

**11.** Composition de test conforme à la revendication 10, dans laquelle ladite seconde enzyme est une oxydase.

**12.** Composition de test conforme à la revendication 11, dans laquelle ladite oxydase est l'une des oxydases énumérées dans la revendication 4.